# EUROPEAN PATENT SPECIFICATION

(11) **EP 4 056 998 B1**
(45) Date of publication and mention of the grant of the patent: **15.07.2026**
(21) Application number: 22160494.5
(22) Date of filing: 07.03.2022
(51) Int. Cl.: G01N 33/18, G01N 21/64, G01N 21/27, C12Q 1/10, G01N 35/02, G01N 35/00

(54) **AUTOMATED AND AUTONOMOUS DEVICE FOR DETECTING THE PRESENCE OF E.COLI AND COLIFORMS IN WATER FOR HUMAN CONSUMPTION**
AUTOMATISIERTE UND AUTONOME VORRICHTUNG ZUM DETEKTIEREN DES VORHANDENSEINS VON E.COLI UND COLIFORMEN IN WASSER FÜR DEN MENSCHLICHEN VERBRAUCH
DISPOSITIF AUTOMATISÉ ET AUTONOME POUR DÉTECTER LA PRÉSENCE D'E.COLI ET DE COLIFORMES DANS L'EAU DESTINÉE À LA CONSOMMATION HUMAINE

(30) Priority: 09.03.2021 CO 21003087
(43) Date of publication of application: 14.09.2022
(73) Proprietor: Excelec International S.A.S., La Estrella, Antioquia (CO)
(72) Inventor: ARANGO URIBE, Juan David, Medellín, Antioquia (CO); ESCUDERO ATEHORTUA, Luis Felipe, Itagui, Antioquia (CO)
(74) Representative: Cueto, Sénida

(56) References cited:
- KR-A- 20150 068 755
- KR-B1- 102 198 400
- US-A- 5 605 812
- US-B2- 8 841 118
- COLIFAST: "Colifast ALARM Monitors the Water Quality in a Clean Water Reservoir", 21 August 2019 (2019-08-21), pages 1 - 1, XP093216613, Retrieved from the Internet <URL:https://www.colifast.no/_files/ugd/b30c09_a8f22425fcd543bb8b60063ee9296e09.pdf> [retrieved on 20241021]

## Description

### FIELD OF THE INVENTION

The present invention refers to an automated and autonomous device for the detection of contaminating bacteria in water, particularly for detecting coliforms and *Escherichia coli,* which does not require an operator and which reports the test results to a remote center.

### BACKGROUND OF THE INVENTION

One of the most used indicators to guarantee the quality of drinking water is the determination of its microbial load, which is generally achieved by detecting the presence of coliforms. These bacteria are useful to indicate the presence of fecal contamination in drinking water, and, therefore, the possibility of the presence of pathogens. In this sense, the detection of *Escherichia coli* is one of the most used indicators in the determination of this type of microbial contamination, being further complemented by the detection of total coliforms, which not only include *Escherichia coli,* which is always found in human and animal feces, but also many other genera, such as *Enterobacter, Citrobacter* and *Serratia,* which are present in the environment and which are not associated with fecal contamination.

*Escherichia coli,* or *E. coli,* belongs to the *Escherichia* genus and it is a well-known member of the Enterobacteriaceae family of bacteria. Enterobacteraceae are commonly known as enteric bacteria, because they can survive in the gastrointestinal tract, which consists of the structures of the digestive system (oral cavity, esophagus, stomach, intestines, rectum, and anus). Other members of the Enterobacteriaceae family include *Klebsiella, Shigella* and *Salmonella.* Some of these organisms are commonly associated with foodborne illnesses or illnesses caused by organisms in the water. The great variety of microorganisms, mainly from the afore-mentioned families, are harmful to human health, which, in extreme cases, can cause hemorrhagic diarrhea, kidney failure, and even death.

Traditionally, the tests for the detection of *E. Coli* and coliforms are carried out in laboratories, in which it is necessary to send the water samples taken directly from the treatment plants, which involves a series of logistical and economic inconveniences, particularly in remote rural areas. Also, this procedure involves a relatively long time, because it is necessary to send and analyze the samples and receive the results of each one, for which there is a risk of supplying water not suitable for human consumption while this entire process is completed. Similarly, the time and conditions of sample transportation can alter the results of the test in the laboratory, generating inconveniences in decision-making in treatment plants. Only in large water treatment plants, mainly in highly populated urban areas, do they have the equipment to carry out the tests on site, but always with equipment attended by specialized personnel for analysis. The patent document KR102198400B1 discloses a method for automatically inspecting coliform groups and E.coli using chromogenic assay, which can prevent, block, and completely sterilize external contamination during an analysis process, and an apparatus thereof. The method comprises the steps of: weighing a sample; adding a test reagent 1 for removing an oxidizing agent to the weighed sample; adding a test reagent 2 for culturing coliform groups and E. coli to the weighed sample; transferring the samples into which the test reagents 1 and 2 are added to a coliform group and E. coli measurement module; culturing the coliform groups and E. coli at a predetermined temperature; measuring transmitted light and fluorescence once every 10 minutes while culturing the coliform groups and E. coli; determining whether the coliform groups and E. coli are positive or negative based on the measured transmitted light and fluorescence and displaying a determination result; calculating and displaying the number of individuals (CFU) when it is determined that the coliform groups and E. coli are positive; and discharging and cleaning the test samples and sterilizing the test apparatus.

Therefore, a device that allows the detection of microorganisms in treatment plants in a frequent, autonomous and affordable manner, with fast and reliable results, is desirable to guarantee an optimal quality of water for human consumption.

### BRIEF DESCRIPTION OF THE INVENTION

In view of the problems identified above, the present invention provides an automated and autonomous device for the detection of microorganisms in water, comprising a flask dispensing module, comprising a flask container and flasks, wherein the flasks contain a dehydrated or hydrated culture medium and a medium to remove chlorides; an injection module that allows adding the water to be analyzed to the flasks; an incubation and detection module that sets the temperature to allow the proliferation of microorganisms, the incubation and detection module comprises a culturing oven, a dark chamber, a UV lamp, a fluorescence sensor, a daylight lamp, and a color sensor;a control module that regulates the currents and the states of each one of the modules; a central module for processing and storing information by means of which it is possible to determine the status of the device, enter orders, and obtain the results of the tests; a communications module that allows the interaction of the flask dispensing module, injection module incubation and detection module, control module, and a central module for processing and storing information; wherein the injection module has means to provide a sterilization fluid through all or some of the components of the module so as to avoid cross-contamination of the samples; wherein the central module for processing and storing information, allows to automatically alert the administrators and those in charge of the device in case of registering the presence of microorganisms harmful to human health and to execute actions on valves and other actuators that prevent the distribution of water not suitable for human consumption.

Thanks to the interaction of the above-mentioned modules, the autonomous detection of potentially dangerous microorganisms for health in drinking water is possible in a quick, affordable and reliable manner.

### DESCRIPTION OF THE DRAWINGS

Figure 1: Block diagram of the device for detecting microorganisms in water according to the invention.
Figure 2: Diagram of the operation flow of the device for detecting microorganisms in water according to the invention.
Figure 3: Diagram of actuators of the device for detecting microorganisms in water according to the invention.
Figure 4a: Front view of a device according to an embodiment of this invention.
Figure 4b: Isometric view of a device according to an embodiment of the present invention.

### DETAILED DESCRIPTION OF THE INVENTION

The device for detecting the presence of contaminating bacteria in drinking water according to the present invention consists of a series of coupled modules, and their interaction makes it possible to prevent the delivery of contaminated water to users of the aqueduct in which the device is used, allowing on-site analysis of samples, without the intervention of specialized personnel.

To carry out this task, the present invention comprises a flask dispensing module; an injection module; an incubation and detection module; a control module; a communications module and a central information processing and storage module, whose components and operation are disclosed below.

The automated and autonomous device for the detection of microorganisms in water for human consumption herein disclosed autonomously performs, without the intervention of operators or analysts, the tests related to the detection of bacteria, such as coliforms and *E. Coli,* and reports the results to the central information processing and storage module, which allows remote consultation by users or managers of the water treatment system.

The central module for processing and storing information (7), allows to automatically alert the administrators and those in charge of the device in case of registering the presence of microorganisms harmful to human health and to execute actions on valves and other actuators that prevent the distribution of water not suitable for human consumption.

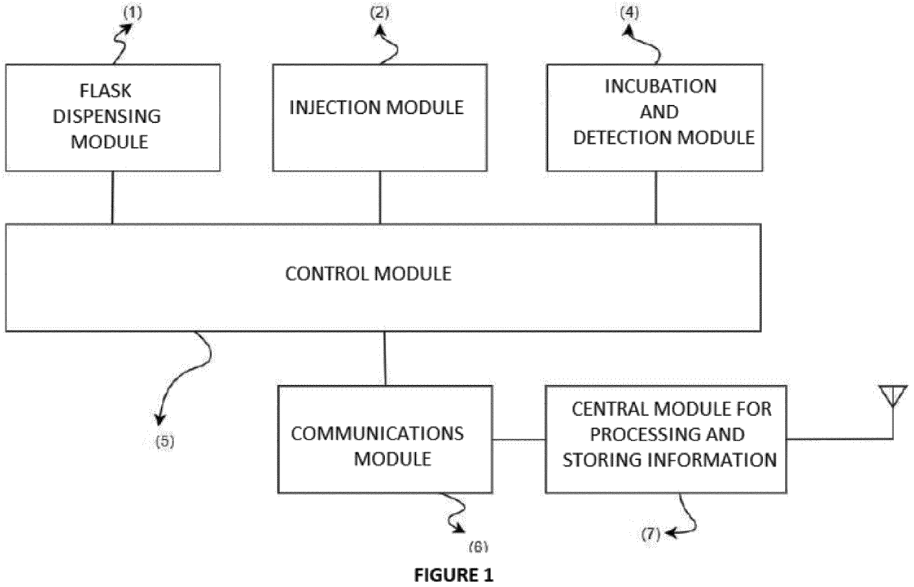

As shown in Figure 1, the device comprises a flask dispensing module (1) in charge of providing flasks containing a sterile culture medium, with the necessary components to allow the proliferation and subsequent detection of the microorganisms to be analyzed, an injection module (2), in charge of introducing the water to be analyzed into the flasks; an incubation and detection module (4) that allows generating the required temperature conditions to allow the proliferation of microorganisms in the culture medium, and their subsequent analysis to check the quality of the water; a control module (5) with the appropriate inputs and outputs for each of the aforementioned modules, which is responsible for regulating the currents and the states of each of the modules of the device; a communications module (6) that allows the interaction of the previous modules with a central module for processing and storing information (7) by means of which it is possible to determine the status of the device, enter orders and obtain the results of the tests.

The communications module (6) connected to the control module (5) is responsible for allowing the entry of orders to be executed by the other modules, as well as for obtaining information on the results that will be communicated to the central module for processing and storing information (7).

In another preferred embodiment, the central module for processing and storing information (7) has a suitable interface for the user to interact with the equipment and record all the information that is relevant to the operator or maintenance technician, such as the calibration of sensors, adjustment of times, temperatures, among others. Once the device is installed, the entire process can be done remotely. Similarly, it is possible to use interfaces that have information storage devices, in order to be able to maintain a database that contains the local information of the latest processes. The interface of the central module for processing and storing information (7) can be selected, without limitation, from among a computer, a tablet, a smartphone or the like.

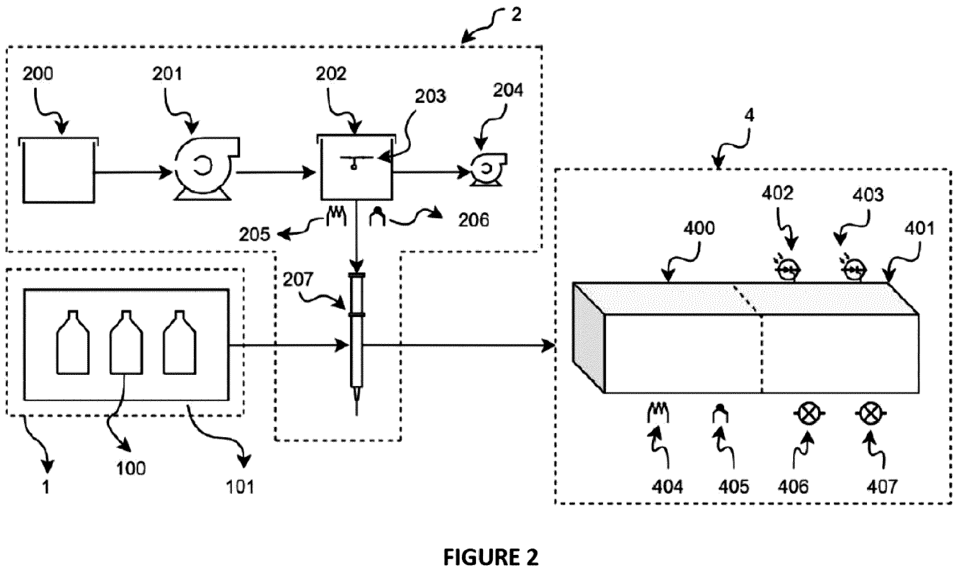

Figure 2 shows a scheme of the flow of operation carried out automatically and without the intervention of operators of the device according to the invention and details, in a more specific manner, the components of the flask dispensing module (1), the injection module (2) and the incubation and detection module (4).

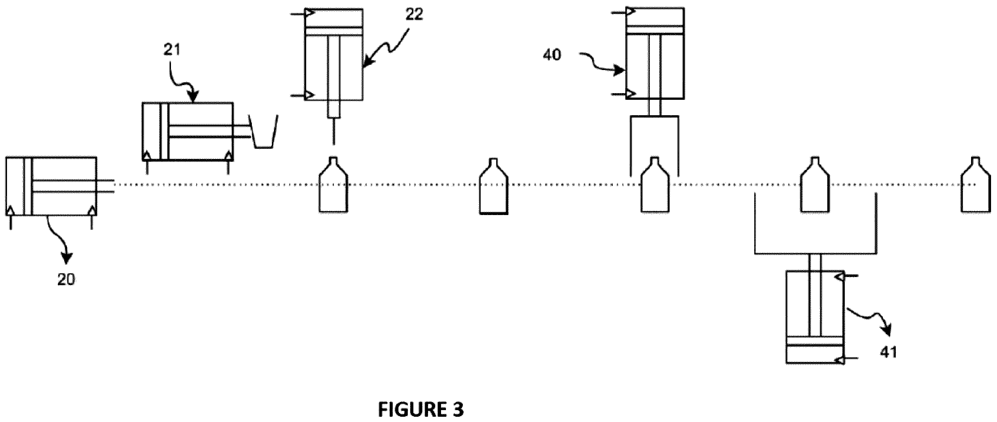

Similarly, Figure 3 shows the diagram of actuators, in which it is observed how the flasks with the culture medium are transported through the entire device. First of all, once the flask is dispensed by the flask dispensing module (1), it is transported to the injection position by means of the transport actuator (20), to allow the injection actuator (22) to move the needle through which the dosing of the water to be analyzed is carried out. Once the water is in the flask, the injection actuator (22) retracts to extract the needle and allow the flask to move to the next point in the process. Subsequently, the transport actuator (20) again moves the flask towards the incubation and detection module (4), wherein the oven actuator (40) positions the oven that will provide the temperature conditions required for the proliferation of possible microorganisms present in the water over the flask. Finally, the oven actuator (40) withdraws the oven to allow the action of the dark chamber actuator (41), which positions the dark chamber over the vial in order to carry out the detection. In cases where sterilization of the system is required, the cleaning actuator (21) can extend a container below the injector to receive the sterilization fluid. In some embodiments of the invention, the transport actuator (20) can be linear or rotary.

As shown in Figure 2, the flask dispensing module (1) provides flasks (100) that are sealed vial-type bottles, so that the injection of the water to be analyzed is possible, maintaining the sterile environment inside. In preferred embodiments, the flasks (100) are sealed with a rubber cap or septa and they contain the necessary reagents to detect the bacteria. In some embodiments of the invention, the flasks (100) contain a hydrated or dehydrated culture medium, a contrast medium and a chloride removal agent, necessary to recover the sublethally damaged cells and guarantee an optimal result of the detection process. In a preferred embodiment of the invention, the chloride removal agent is selected from sulfur dioxide or sodium thiosulfate, and the culture medium is hydrated.

In some embodiments of the invention, the flasks (100) contain a dehydrated culture medium; in these embodiments, the device of the invention further includes an agitation module between the injection module (2) and the incubation and detection module (4), in which the homogenization of the sample is carried out, such that an intimate contact is obtained between the water to be analyzed and the reagents that will allow the identification of the contaminating bacteria.

According to the invention, the flasks (100) are stored in the flask container (101), where enough flasks (100) are stacked so that they are available for a considerable amount of time. In one embodiment of the invention, the flask container (101) contains between 1 and 50 flasks (100), more preferably between 15 and 30 flasks (100). The flasks go down the stack by gravity to where there is a geared motor with a wheel that allows the flasks to be dispensed one by one. Once the flask is dispensed, it must be placed in a vertical position, and it is taken by the transport actuator (20) to the injection position.

In an embodiment of the invention shown in Figures 2 and 3, the injection module has a transport actuator (20) that is responsible for taking the flask provided by the bottle dispensing module (1) to the injection position. The water supplied to the subscribers to the supply of the treatment plant is stored in the tank (200), from which a sample will be taken to carry out the subsequent analysis. Once there is enough water in the tank (200), the pump (201) is activated to fill the injection tank (202), which has a tank level sensor (203), which allows determining the volume of the injection tank (202) and deactivating the flow of water through the pump when reaching said volume. The injection actuator (22) ensures that the injector needle (207) pierces the bottle cap to allow water to enter. Once the injection tank (202) is full and the needle is inserted into the flask, the injection pump (204) turns on. The volume required depends on the type of flask and the characteristics of the culture and contrast medium thereof. In general, the volume of water is between 50 mL and 250 mL. The water volume is confirmed with a "non-contact" type sensor or similar that is outside the flask. When the correct level is reached, the injection pump (204) turns off and the injection actuator (22) withdraws the needle from the flask.

Since consecutive tests are performed, the device should have means to provide a sterilization fluid for the injection module (2) to avoid cross-contamination of samples. Accordingly, an embodiment of the device according to the present invention has a sterilization resistance (205) in the injection tank (202) which can heat a sterilization fluid to a given temperature, which can be the boiling temperature of the sterilization fluid, controlled by the sterilization temperature sensor (206). Once the sterilization temperature is reached, the sterilization fluid is allowed to pass through the different components of the injection module (2). In a preferred embodiment, the sterilized components are the injection tank (202), the injector (207) and the injection pump (204). In case sterilization is required, the cleaning actuator (21) positions a drainage container below the injector (207) to collect the sterilization fluid, and, subsequently, it is possible to make the sterilization fluid pass through the components to be sterilized. This guarantees total sterility of the tanks, hoses and the injection needle that connect the entire system for subsequent analysis. In a preferred embodiment, the sterilization fluid is selected from water.

Subsequently, the flask is positioned in the incubation and detection module (4), which has an oven actuator (40) that positions the culturing oven (400) over the flask in order to keep it at the culture temperature for the necessary time to allow the proliferation of microorganisms of interest. To this end, in one embodiment of the device, it comprises an oven resistance (404) and an oven thermocouple (405), which are responsible for heating and controlling the temperature, respectively. In embodiments of the invention, the incubation temperature is between 25°C and 50°C, more preferably between 35°C and 44°C, and the incubation time is between 8 and 48 hours, more preferably between 12 and 24 hours. Once the incubation stage is concluded, the oven actuator (40) withdraws the culturing oven (400), to allow the dark chamber actuator (41) to position the dark chamber (401) necessary for the detection stage.

During the detection stage, thanks to the presence of a chromofluorogenic contrast agent in the flasks (100), the spectrophotometric determination of the microorganisms of interest is possible in the incubation and detection module (4). The detection section of the incubation and detection module (4) has a UV lamp (402) and a daylight lamp (403). The UV lamp (402) can be selected from any type, including low, medium, high pressure and ultra-high pressure, which are made of various materials, preferably mercury (Hg). Spectral calibration lamps, electrodeless lamps and the like can also be used. UV-A lamps, which provide radiation from about 315nm to about 400nm, are preferred. In preferred embodiments, the UV lamp (402) comprises 18W ultraviolet light bulbs that emit 366nm wavelength light, and the daylight lamp (403) comprises 9W bulbs that emit 6500K light. The UV lamp (402) is initially turned on to allow fluorescence sensor (406) to determine the presence or absence of fluorescence in the sample. Subsequently, the UV lamp (402) is turned off and the daylight lamp (403) is turned on, so that the color sensor (407) determines the presence or absence of blue coloration in the sample. If fluorescence is detected in the sample through the fluorescence sensor (406), the result would be positive for *E.Coli.* Similarly, if the color sensor (407) detects that the sample has turned blue, the test is considered positive for coliform bacteria. In preferred embodiments of the invention, the culturing oven (400) and the dark chamber (401) are contained in a single unit that performs both functions, and therefore only one actuator is required to position this unit over the flask.

According to a preferred embodiment of the invention, in case the tests with both the UV lamp (402) and the daylight lamp (403) are negative, the dark chamber actuator (41) is removed, allowing the oven actuator (40) to, once again, position itself over the sample to proceed with an additional incubation period of at least 24 hours, after which the detection is repeated. If the result is negative once again, the water is considered fit for human consumption. Once this step is finished, the transport actuator (20) again pushes the flask to the end of the platform to discard it. The test results will be displayed on the user interface, which is located in the central information processing and storage module (7) and are accessible both on site and remotely.

Since the result could have been positive, the sterilization of the injection tank (202), the injection pump (204) and the injector (207) is essential to prevent subsequent tests from being affected, which is why the above-mentioned sterilization stage must be carried out, once the end of the incubation and detection stage is reached.

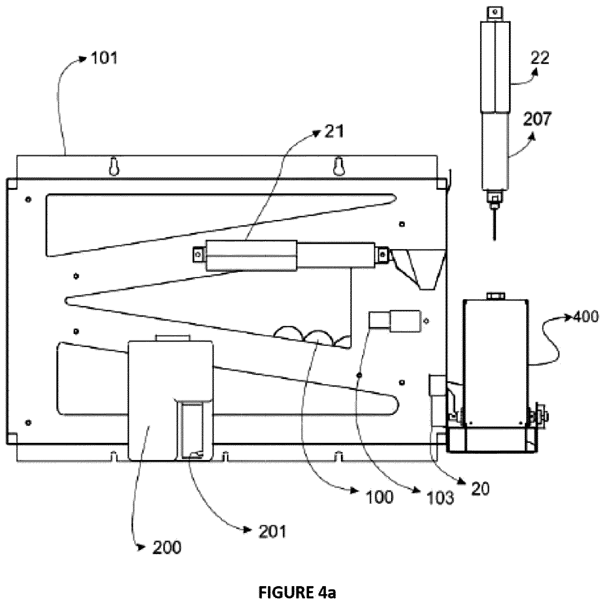

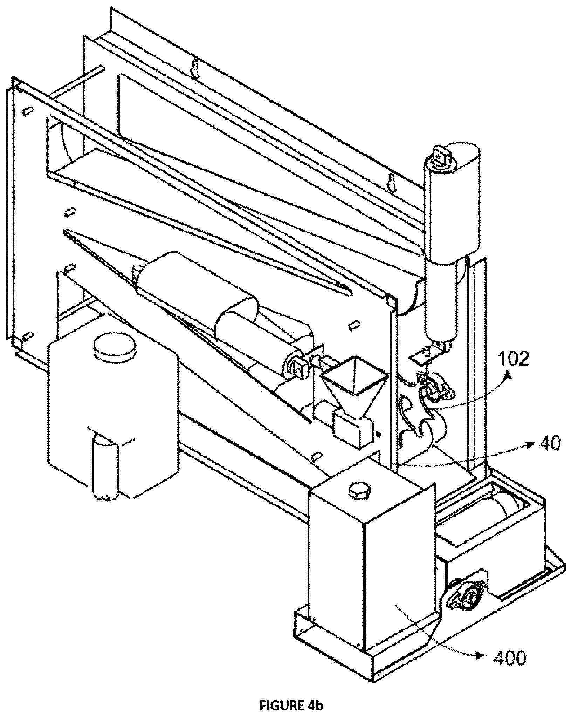

Figure 4a shows a front view of an embodiment according to some embodiments of the present invention. It also shows the geared motor (103) in charge of providing the flasks (100) one by one thanks to the action of the toothed wheel or sprocket (102) shown in Figure 4b. This geared motor rotates the wheel at a speed of approximately 2 RPM, so that the dispensing of the flasks (100) is controlled in a precise manner.

Figure 4b shows an isometric view of an embodiment of the device according to the present invention, which represents the mode in which the culturing oven (400) acts as an oven and as a dark chamber, performing both the incubation and detection functions. In this particular embodiment, since the culturing oven (400) and the dark chamber (401) are contained in a single unit that performs both functions, only one actuator is required to position this unit over the flask.

On the other hand, according to the invention, the communications module (6) can use wireless or wired channels to communicate information and send orders to or from the flask dispensing module (1), the injection module (2) and the incubation and detection module (4). Communications as such can be by cell phone, fiber optics, local network by Ethernet, radio frequency such as those accepted by the IEEE802.15.4 standard, among others. The use of one type or another depends on the local conditions and the technology available in the place where the device will work.

According to embodiments of the device of the invention, the vial dispensing (1), injection (2) and incubation and detection (4) modules are connected to the control module (5), which has a microcontroller card, in charge of controlling the actuators, resistors, motors, lamps, sensors and other systems involved in the operation of the device.

Power cards are connected to the microcontroller card, and they have relays for handling high-current loads such as actuators and resistors. Every actuator must have two relays, which are required to have the following three states: paused, extending and retracting. The resistors, motors, pumps, lamps, among others, also have relays for their operation. There are analog inputs for the temperature sensors and the light sensors, as well as digital inputs for limit switches and level sensors. The currents and voltages required by the logic circuits and power circuits are supplied by a suitable power source.

## Claims

1. An automated and autonomous device for the detection of microorganisms in water, comprising:
a flask dispensing module (1), comprising a flask container (101) and flasks (100), wherein the flasks contain a dehydrated or hydrated culture medium and a medium to remove chlorides;
an injection module (2) that allows adding the water to be analyzed to the flasks (100);
an incubation and detection module (4) that sets the temperature to allow the proliferation of microorganisms, the incubation and detection module (4) comprises a culturing oven (400), a dark chamber (401), a UV lamp (402), a fluorescence sensor (406), a daylight lamp (403), and a color sensor (407);
a control module (5) that regulates the currents and the states of each one of the modules;
a central module for processing and storing information (7) by means of which it is possible to determine the status of the device, enter orders, and obtain the results of the tests;
a communications module (6) that allows the interaction of the flask dispensing module (1), injection module (2) incubation and detection module (4), control module (5), and a central module for processing and storing information (7);
wherein the injection module (2) has means to provide a sterilization fluid through all or some of the components of the module so as to avoid cross-contamination of the samples;
wherein the central module for processing and storing information (7), allows to automatically alert the administrators and those in charge of the device in case of registering the presence of microorganisms harmful to human health and to execute actions on valves and other actuators that prevent the distribution of water not suitable for human consumption.

2. The device according to Claim 1, wherein the microorganisms are *Escherichia coli* and coliforms;

3. The device according to Claim 1, wherein an additional agitation module is included when the flask has a dehydrated culture medium.

4. The device according to Claim 1, wherein the injection module (2) comprises an injector (207), an injection tank (202), and an injection pump (204).

5. The device according to Claim 4, wherein the injection module (2) also has a no-contact flask level sensor (209) to determine the volume of water in the flask.

6. The device according to Claim 4, wherein the injection tank (202) of the injection module (2) further comprises a sterilization resistance (205) and a sterilization temperature sensor (206) to sterilize the injection module (2).

7. The device according to Claim 6, wherein the injection module (2) has means to provide a sterilization fluid to sterilize the injection tank (202), the injection pump (204) and the injector (207).

8. The device according to Claim 7, wherein the injection module (2) is suitable to provide boiling water as sterilization fluid.

9. The device according to Claim 1, wherein the culturing oven (400) comprises an oven resistor (404) and an oven thermocouple (405).

10. The device according to Claim 1 , wherein the culturing oven (400) and the dark room (401) are in a unit that performs the two functions.

11. The device according to Claim 1, wherein the control module (5) comprises power cards with relays and analog inputs.

12. The device according to Claim 1, wherein the communications module (6) can be wireless or wired.

13. The device according to Claim 12, wherein the communications module (6) is selected from the group comprising cellular networks, fiber optics, local Ethernet network, and radio frequency communications, such as those covered by the IEEE802.15.4 standard.

## Patentansprüche

1. Eine automatisierte und autonome Vorrichtung zum Nachweis von Mikroorganismen in Wasser, umfassend:
ein Kolbenausgabemodul (1), das einen Kolbenbehälter (101) und Kolben (100) umfasst, wobei die Kolben ein dehydriertes oder hydratisiertes Kulturmedium und ein Medium zur Entfernung von Chloriden enthalten;
ein Injektionsmodul (2), das das Hinzufügen des zu analysierenden Wassers zu den Kolben (100) ermöglicht;
ein Inkubations- und Detektionsmodul (4), das die Temperatur so einstellt, dass sich Mikroorganismen vermehren können, wobei das Inkubations- und Detektionsmodul (4) einen Kultivierungsofen (400), eine Dunkelkammer (401), eine UV-Lampe (402), einen Fluoreszenzsensor (406), eine Tageslichtlampe (403) und einen Farbsensor (407) umfasst;
ein Steuermodul (5), das die Ströme und die Zustände jedes einzelnen Moduls regelt;
ein Zentralmodul zur Verarbeitung und Speicherung von Informationen (7), mittels dessen es möglich ist, den Status des Geräts zu ermitteln, Befehle einzugeben und die Testergebnisse abzurufen;
ein Kommunikationsmodul (6), das die Interaktion zwischen dem Kolbenausgabemodul (1), dem Injektionsmodul (2), dem Inkubations- und Detektionsmodul (4), dem Steuermodul (5) und einem zentralen Modul zur Verarbeitung und Speicherung von Informationen (7) ermöglicht;
wobei das Injektionsmodul (2) Mittel aufweist, um eine Sterilisationsflüssigkeit durch alle oder einige der Komponenten des Moduls zu leiten, um eine Kreuzkontamination der Proben zu vermeiden;
wobei das zentrale Modul zur Verarbeitung und Speicherung von Informationen (7) es ermöglicht, die Administratoren und die für das Gerät Verantwortlichen automatisch zu alarmieren, falls das Vorhandensein von für die menschliche Gesundheit schädlichen Mikroorganismen registriert wird, und Maßnahmen an Ventilen und anderen Stellgliedern auszuführen, die die Verteilung von Wasser verhindern, das nicht für den menschlichen Verzehr geeignet ist.

2. Vorrichtung nach Anspruch 1, wobei die Mikroorganismen *Escherichia coli* und Coliforme sind;

3. Vorrichtung nach Anspruch 1, wobei ein zusätzliches Rührmodul vorgesehen ist, wenn der Kolben ein dehydriertes Kulturmedium enthält.

4. Vorrichtung nach Anspruch 1, wobei das Injektionsmodul (2) einen Injektor (207), einen Injektionsbehälter (202) und eine Injektionspumpe (204) umfasst.

5. Vorrichtung nach Anspruch 4, wobei das Injektionsmodul (2) zudem einen berührungslosen Kolbenfüllstandssensor (209) aufweist, um das Wasservolumen im Kolben zu bestimmen.

6. Vorrichtung nach Anspruch 4, wobei der Injektionsbehälter (202) des Injektionsmoduls (2) ferner einen Sterilisationswiderstand (205) und einen Sterilisationstemperatursensor (206) umfasst, um das Injektionsmodul (2) zu sterilisieren.

7. Vorrichtung nach Anspruch 6, wobei das Injektionsmodul (2) Mittel zum Bereitstellen einer Sterilisationsflüssigkeit aufweist, um den Injektionsbehälter (202), die Injektionspumpe (204) und den Injektor (207) zu sterilisieren.

8. Vorrichtung nach Anspruch 7, wobei das Injektionsmodul (2) dazu geeignet ist, kochendes Wasser als Sterilisationsflüssigkeit bereitzustellen.

9. Vorrichtung nach Anspruch 1, wobei der Kultivierungsofen (400) einen Ofenwiderstand (404) und ein Ofenthermoelement (405) umfasst.

10. Vorrichtung nach Anspruch 1, wobei der Kultivierungsofen (400) und die Dunkelkammer (401) in einer Einheit angeordnet sind, die beide Funktionen erfüllt.

11. Vorrichtung nach Anspruch 1, wobei das Steuermodul (5) Leistungsplatinen mit Relais und analogen Eingängen umfasst.

12. Vorrichtung nach Anspruch 1, wobei das Kommunikationsmodul (6) drahtlos oder kabelgebunden sein kann.

13. Vorrichtung nach Anspruch 12, wobei das Kommunikationsmodul (6) aus der Gruppe ausgewählt ist, die Mobilfunknetze, Glasfasernetzwerke, lokale Ethernet-Netzwerke und Funkfrequenzkommunikation umfasst, wie sie beispielsweise durch den Standard IEEE802.15.4 abgedeckt sind.

## Revendications

1. Un dispositif automatisé et autonome pour la détection de micro-organismes dans l'eau, comprenant:
un module de distribution de flacons (1), comprenant un récipient à flacons (101) et des flacons (100), dans lequel les flacons contiennent un milieu de culture déshydraté ou hydraté et un milieu pour éliminer les chlorures;
un module d'injection (2) qui permet d'ajouter l'eau à analyser aux flacons (100);
un module d'incubation et de détection (4) qui définit la température permettant la prolifération de micro-organismes, le module d'incubation et de détection (4) comprenant une étuve de culture (400), une chambre noire (401), une lampe UV (402), un capteur de fluorescence (406), une lampe à lumière du jour (403) et un capteur de couleur (407);
un module de contrôle (5) qui régule les courants et les états de chacun des modules ;
un module central pour traiter et stocker les données (7) grâce auquel on peut déterminer l'état du dispositif, saisir des commandes et obtenir les résultats des tests;
un module de communications (6) qui permet l'interaction du module de distribution de flacons (1), le module d'injection (2), le module d'incubation et de détection (4), le module de contrôle (5), et un module central pour le traitement et le stockage des données;
dans lequel le module d'injection (2) peut administrer un fluide de stérilisation par l'intermédiaire de tous les composants du module ou d'une partie d'entre eux de manière à éviter la contamination croisée des échantillons (7);
dans lequel le module d'injection (2) dispose des moyens de fournir un fluide stérilisant à travers tous les composants du module ou une partie d'entre eux de manière à éviter une contamination croisée des échantillons;
dans lequel le module central de traitement et de stockage des données (7) permet de prévenir automatiquement les administrateurs et les responsables du dispositif en cas de détection de la présence de micro-organismes dangereux pour la santé humaine et d'intervenir sur les vannes et tout autre actionneur évitant la distribution d'eau impropre à la consommation humaine.

2. Le dispositif selon la revendication 1, dans lequel les micro-organismes sont des *Escherichia coli* et des coliformes.

3. Le dispositif selon la revendication 1, dans lequel on inclut un module d'agitation additionnel lorsque le flacon contient un milieu de culture déshydraté.

4. Le dispositif selon la revendication 1, dans lequel le module d'injection (2) comprend un injecteur (207), un réservoir d'injection (202), et une pompe d'injection (204).

5. Le dispositif selon la revendication 4, dans lequel le module d'injection (2) présente également un capteur de niveau de flacon sans contact (209) pour déterminer le volume d'eau à l'intérieur du flacon.

6. Le dispositif selon la revendication 4, dans lequel le réservoir d'injection (202) du module d'injection (2) comprend en outre une résistance de stérilisation (205) et un capteur de température de stérilisation (206) pour stériliser le module d'injection (2).

7. Le dispositif selon la revendication 6, dans lequel le module d'injection (2) a les moyens de fournir un fluide stérilisant pour stériliser le réservoir d'injection (202), la pompe d'injection (204) et l'injecteur (207).

8. Le dispositif selon la revendication 7, dans lequel le module d'injection (2) convient pour fournir de l'eau bouillante comme fluide de stérilisation.

9. Le dispositif selon la revendication 1, dans lequel l'étuve de culture (400) comprend une résistance d'étuve (404) et un thermocouple d'étuve (405).

10. Le dispositif selon la revendication 1, dans lequel le four de laboratoire (400) et la chambre noire (401) sont dans une unité qui effectue les deux fonctions.

11. Le dispositif selon la revendication 1, dans lequel le module de contrôle (5) comprend des cartes d'alimentation avec des relais et des entrées analogues.

12. Le dispositif selon la revendication 1, dans lequel le module de communication (6) peut être sans fil ou filaire.

13. Le dispositif selon la revendication 12, dans lequel le module de communications (6) est sélectionné dans le groupe comprenant des réseaux cellulaires, des fibres optiques, un réseau Ethernet local et des communications de radiofréquence, tels que ceux couverts par le IEEE802.15.4 standard.
